(19) Europäisches Patentamt
European Patent Office
Office européen des brevets

(11) Veröffentlichungsnummer: **0 011 221**
**B1**

(12) # EUROPÄISCHE PATENTSCHRIFT

(45) Veröffentlichungstag der Patentschrift: **16.03.83**

(51) Int. Cl.[3]: **C 07 D 277/50,** A 01 N 47/24

(21) Anmeldenummer: **79104345.8**

(22) Anmeldetag: **06.11.79**

(54) **Carbamoyl-oxime, Verfahren zu deren Herstellung, Schädlingsbekämpfungsmittel enthaltend solche Verbindungen sowie Verwendung solcher Verbindungen als Schädlingsbekämpfungsmittel.**

(30) Priorität: **09.11.78 CH 11531/78**
**24.08.79 CH 7728/79**

(43) Veröffentlichungstag der Anmeldung: **28.05.80 Patentblatt 80/11**

(45) Bekanntmachung des Hinweises auf die Patenterteilung: **16.03.83 Patentblatt 83/11**

(84) Benannte Vertragsstaaten: **AT BE CH DE FR GB IT LU NL SE**

(56) Entgegenhaltungen:
**DE-A-2 222 464**
**FR-A-2 136 055**
**FR-A-2 317 295**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Winternitz, Paul, Dr., Am Pfisterhölzli 50 Greifensee, CH-8606 Nänikon-Greifensee (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 011 221 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Carbamoyl-oxime, Verfahren zu deren Herstellung, Schädlingsbekämpfungsmittel, enthaltend solche Verbindungen sowie Verwendung solcher Verbindungen als Schädlingsbekämpfungsmittel

Die Erfindung betrifft Carbamoyl-oxime der allgemeinen Formel

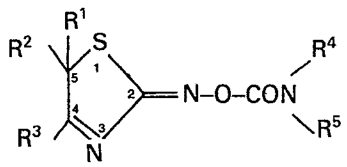 I

worin $R^1$ und $R^3$ $C_{1-5}$-Alkyl, $C_{2-5}$-Alkenyl, $C_{3-6}$-Cycloalkyl oder Phenyl oder Naphthyl bedeuten, wobei der Phenyl- oder Naphthylrest gegebenenfalls mit einem Halogenatom substituiert sein kann, $R^2$ $C_{1-5}$-Alkyl, $R^4$ $C_{1-5}$-Alkyl oder $C_{3-6}$-Cycloalkyl und $R^5$ Wasserstoff, $C_{1-5}$-Alkyl, $C_{2-6}$-Alkylcarbonyl oder mit Halogen substituiertes $C_{2-6}$-Alkylcarbonyl bedeuten, und Säureadditionssalze dieser Verbindungen.

Die Verbindungen der Formel I und deren Salze sind Schädlingsbekämpfungsmittel und eignen sich insbesondere zur Bekämpfung von Insekten. Somit umfasst die Erfindung auch Schädlingsbekämpfungsmittel, die eine oder mehrere Verbindungen der allgemeinen Formel I bzw. Salze davon enthalten, Verfahren zur Herstellung derartiger Mittel sowie deren Verwendung.

Die Deutsche Offenlegungsschrift 2222464 beschreibt u. a. pestizide 2-Carbamoyloximino-4-thiazolidinone. Von diesen bekannten Verbindungen unterscheiden sich die erfindungsgemässen im wesentlichen dadurch, dass sie einen 4,5,5-trisubstituierten Thiazolinkern, d. h. einen Kern der Formel

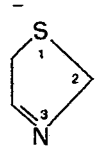

anstatt eines gegebenenfalls 3,5,5-trisubstituierten 4-Thiazolidinonkerns, d. h. eines Kerns der Formel

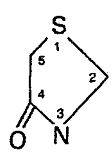

aufweisen.

Die Erfindung betrifft ferner Verfahren zur Herstellung der Verbindungen der Formel I und deren Salze. Das erfindungsgemässe Verfahren ist dadurch gekennzeichnet, dass man ein Oxim der Formel

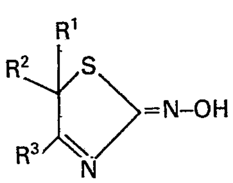 II

worin $R^1$, $R^2$ und $R^3$ die oben angegebene Bedeutung besitzen,

a) mit einem Isocyanat der Formel

$$R^4{-}N{=}C{=}O \quad III$$

in welcher $R^4$ die oben angegebene Bedeutung hat, in Gegenwart eines Verdünnungsmittels und gegebenenfalls in Gegenwart eines Katalysators umsetzt, oder

b) mit einem Carbamoylhalogenid der Formel

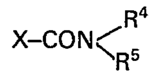 IV

in welcher $R^4$ und $R^5$ die oben angegebene Bedeutung haben und X ein Halogenatom bedeutet, in Gegenwart eines Verdünnungsmittels und eines säurebindenden Mittels umsetzt, und erwünschtenfalls eine erhaltene Verbindung der Formel I mit einer Säure in ein Säureadditionssalz überführt.

Der Ausdruck «$C_{1-5}$-Alkyl» umfasst – wenn nicht ausdrücklich anderweitig definiert – sowohl geradkettige als auch verzeigte Kohlenwasserstoffradikale mit 1–5 Kohlenstoffatomen, wie Methyl, Äthyl, Propyl, Isopropyl und dergleichen. Dies gilt auch für $C_{1-5}$-Alkylgruppen enthaltende Reste wie $C_{2-6}$-Alkylcarbonyl und halogensubstituiertes $C_{2-6}$-Alkylcarbonyl. Der Ausdruck «$C_{2-5}$-Alkenyl» umfasst sowohl geradkettige als auch verzweigte Kohlenwasserstoffradikale mit 2–5 Kohlenstoffatomen. Als Beispiele seien genannt: Vinyl, Allyl, Butenyl und Pentenyl. Der Ausdruck «Halogen» umfasst die vier Halogenatome Fluor, Chlor, Brom und Jod, wenn nicht ausdrücklich anderweitig angegeben.

Als Säureadditionssalze der Verbindungen der Formel I, die aber, verglichen mit den Basen I, von geringerer Bedeutung sind, kommen physiologisch verträgliche Salze in Frage. Hierzu gehören vorzugsweise Salze der Verbindungen I mit Halogenwasserstoffsäuren, wie z. B. der Chlorwasserstoffsäure und der Bromwasserstoffsäure, ferner mit Phosphorsäure, Salpetersäure, ausserdem mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salicylsäure, Sorbinsäure und Milchsäure, und schliesslich Sulfonsäure, wie die 1,5-Naphthalin-disulfonsäure.

Die Erfindung betrifft auch die syn- und anti-Formen der Verbindungen der Formel I.

Verbindungen der Formel I, worin $R^1$, $R^2$ und $R^3$ Alkyl mit 1–3 Kohlenstoffatomen bedeuten, sind bevorzugt.

Weiter sind Verbindungen der Formel I bevorzugt, worin $R^4$ Alkyl mit 1–3 Kohlenstoffatomen und $R^5$ Wasserstoff bedeuten.

Besonders bevorzugte Verbindungen sind:

2-Oxo-4,5,5-trimethyl-3-thiazolin-O-(methylcarbamoyl)-oxim,

2-Oxo-4,5-dimethyl-5-äthyl-3-thiazolin-O-(methylcarbamoyl)-oxim, und

2-Oxo-4,5-diäthyl-5-methyl-3-thiazolin-O-(methylcarbamoyl)-oxim.

Der Reaktionsverlauf lässt sich bei Verwendung der erwähnten Oxime der Formel II, Isocyanate der Formel III und Carbamoylhalogenide der Formel IV als Ausgangsstoffe durch folgendes Formelschema wiedergeben (Verfahrensvariante a):

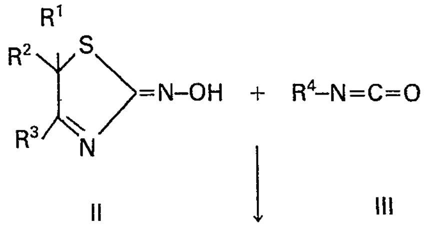

II III

I

(Verfahrensvariante b):

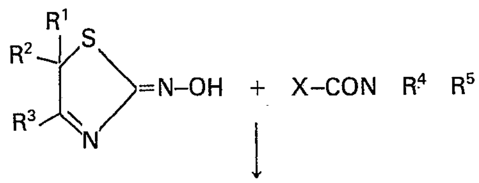

II I IV

Die als Ausgangsprodukte verwendeten Oxime sind durch die Formel II definiert. In Formel II steht $R^1$, $R^2$ und $R^3$ vorzugsweise für geradkettiges oder verzweigtes Alkyl mit 1–3 Kohlenstoffatomen.

Die Oxime der Formel II sind bislang noch nicht bekannt; sie können aber auf einfache Weise hergestellt werden, indem man ein α-Thiocyanatoketon der Formel

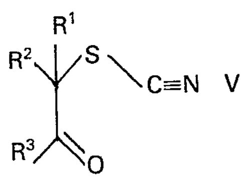

V

in welcher $R^1$, $R^2$ und $R^3$ die oben angegebene Bedeutung haben,
mit Hydroxylamin in Gegenwart eines Lösungsmittels, vorzugsweise eines Alkohols bzw. eines wässrigen Alkohols, bei Temperaturen zwischen 5 und 100°C, vorzugsweise zwischen 25 und 40°C, umsetzt. Dabei wird das Hydroxylamin vorzugsweise in Form eines Salzes, insbesondere als Hydrochlorid, in Gegenwart eines Säurebindemittels, wie beispielsweise Natriumhydrogencarbonat oder Natriumacetat, eingesetzt. Die Isolierung der Verbindungen der Formel II erfolgt dadurch, dass man den während der Umsetzung entstandenen Feststoff nach Abdestillieren des Lösungsmittels mit Wasser digeriert, abfiltriert, trocknet und gegebenenfalls umkristallisiert, oder in einem geeigneten Lösungsmittel aufnimmt, mit Wasser wäscht, über Natriumsulfat trocknet und nach Abdestillieren des Lösungsmittels umkristallisiert oder chromatographisch reinigt.

Die als Ausgangsstoffe verwendeten α-Thiocyanatoketone der Formel V sind zum Teil bekannt und lassen sich nach üblichen Verfahren herstellen, z. B. durch Austausch des Halogenatomes in α-Halogenketonen gegen die Thiocyanatogruppe mittels Metallrhodaniden in einem geeigneten Lösungsmittel wie z. B. Aceton oder Dimethylformamid. Die benötigten α-Halogenketone sind zum Teil bekannt und können nach üblichen Verfahren, wie z. B. durch Halogenierung der Carbonylverbindungen mit Brom in Dioxan, oder N-Bromsuccinimid in Tetrachlorkohlenstoff, hergestellt werden.

Die weiterhin zu dem erfindungsgemässen Herstellungsverfahren benötigten Isocyanate bzw. Carbamoylhalogenide sind durch die Formeln III bzw. IV definiert. In diesen Formeln steht $R^4$ vorzugsweise für Alkyl mit 1–3 Kohlenstoffatomen. $R^5$ steht vorzugsweise für Wasserstoff oder Alkyl mit 1–3 Kohlenstoffatomen, insbesondere für Methyl.

Die Isocyanate der Formel III sind bekannt oder lassen sich nach allgemein üblichen und bekannten Verfahren herstellen, z. B. durch Umsetzung von Aminen mit Phosgen und anschliessendes Erhitzen.

Die Carbamoylhalogenide der Formel IV sind bekannt oder lassen sich nach allgemein üblichen und bekannten Verfahren herstellen, z. B. durch Umsetzung von Aminen mit Phosgen.

Als Verdünnungsmittel kommen für die Umsetzung gemäss Verfahrensvariante a) vorzugsweise alle inerten organischen Lösungsmittel in Frage. Hierzu gehören vorzugsweise Äther wie 1,2-Dimethoxyäthan, Tetrahydrofuran oder Dioxan; Ketone wie Diäthylketon, insbesondere Aceton und Methyläthylketon; Nitrile wie Propionitril, insbesondere Acetonitril; Formamide wie insbesondere Dimethylformamid; und halogenierte Kohlenwasserstoffe, wie Methylenchlorid, Tetrachlorkohlenstoff oder Chloroform.

Als Katalysatoren können beim Verfahren a) vorzugsweise verwendet werden: tertiäre Base, wie Triäthylamin, Pyridin, 1,4-Diazabicyclo[2.2.2]octan; und Zinn-organische Verbindungen, wie Dibutylzinndiacetat.

Die Reaktionstemperaturen können bei der Durchführung des Verfahrens a) in einem grösseren Bereich variiert werden. Im allgemeinen arbeitet man zwischen 0 und 100°C, vorzugsweise zwischen 30 und 85°C. Bei der Anwesenheit eines Lösungsmittels wird zweckmässigerweise beim Siedepunkt des jeweiligen Lösungsmittels gearbeitet.

Bei der Durchführung der Verfahrensvariante a) arbeitet man vorzugsweise mit einem Überschuss an Isocyanat. Zur Isolierung der Verbindungen der Formel I wird das Lösungsmittel abdestilliert und der Rückstand nach üblichen Methoden aufgearbeitet.

Für die Durchführung gemäss Verfahrensvariante b) kommen als Verdünnungsmittel vorzugsweise alle inerten organischen Lösungsmittel in Frage. Hierzu gehören vorzugsweise die bei Verfahren a) aufgezählten Lösungsmittel.

Die Umsetzung nach Verfahrensvariante b) wird in Gegenwart eines Säurebinders vorgenommen. Man kann alle üblicherweise verwendbaren anorganischen und organischen Säurebinder zugeben. Hierzu gehören vorzugsweise Alkalicarbonate, wie beispielsweise Natriumcarbonat, Kaliumcarbonat und Natriumhydrogencarbonat; ferner niedere tertiäre Alkylamine, Cycloalkylamine oder Arylalkylamine, wie beispielsweise Triäthylamin und Dimethylbenzylcyclohexylamin; weiterhin Pyridin und Diazabicyclooctan.

Die Reaktionstemperaturen der Verfahrensvariante b) können in einem grösseren Bereich variiert werden. Im allgemeinen arbeitet man zwischen 0 und 100°C, vorzugsweise zwischen 0 und 40°C.

Bei der Durchführung des Verfahrens b) setzt man vorzugsweise auf 1 Mol der Verbindung der Formel II 1 bis 2 Mol an Carbamoylhalogenid der Formel IV ein. Es hat sich als vorteilhaft erwiesen, den Säurebinder in geringem Überschuss (bis ca. 30 Gew.-%) einzusetzen. Die Isolierung der Verbindungen der Formel I erfolgt in üblicher Weise.

Die Überführung einer erhaltenen Verbindung der Formel I mit einer Säure in ein Säureadditionssalz erfolgt in an sich bekannter Weise.

Die Verbindungen der Formel I sind ganz allgemein als Pestizide von Wert. Sie zeigen sich besonders wertvoll zur Bekämpfung von Insekten, Milben und Nematoden, insbesondere gegen

– Coleoptera, wie z. B. Epilachna spp., Leptinotarsa decemlineata, Anthonomus spp., Conotrachelus nenuphar, Lema spp., Lissorhoptrus cryzaephilus, Phyllotreta spp., Psylliodes chrysocephala, Meligethes aenus, Ceutorrhynchus assimilis, Agriotes spp., Melolontha melolontha, Diabrotica spp.
– Lepidoptera, wie z. B. Laspeyresia spp., Adoxophyes orana, Tortrix viridana, Cheimatobia brumata, Lyonetia clerkella, Operophtera brumata, Lithocolletis blancardella, Porthetria dispar, Mamestra brassicae, Agrotis segetum, Plutella spp., Pieris brassicae, Choristoneura fumiferana, Heliothis spp. Spodoptera spp., Pectinophora gossipiella, Chilo spp., Ostrinia nubilalis, Clysia ambiguella, Lobesia botrana.
– Diptera, wie z. B. Drosophila melanogaster, Ceratitis spp., Oscinella frit, Dacus spp., Rhagoletis spp., Leatherjacket spp.
– Homoptera, d. h. Blattläuse, wie z. B. Aphis fabae, Myzus persicae und weitere Arten dieser Gattungen, Rhophalosiphon spp., Schizaphis spp., Dysaphis spp., Eriosoma spp., Macrosiphum spp., Adelges spp., Sitobion avenae, Metopolophium dirhodum sowie Schild- und Schmierläuse, wie z. B. Aspidiotus spp., Saissetia spp., Quadraspidiotus perniciosus, Aonidiella aurantii, Coccus spp., Lepidosaphes spp., Planococcus spp., Pseudecoccus spp., Ceroplaster spp., Icerya purchasi, Chrysomphalus spp., Parlatoria spp., sowie Zikaden, wie z. B. Nephotettix spp., Laodelphax spp., Nilaparvata spp., Sogatella spp., Erythroneura spp.
– Aleyrodidae, wie z. B. Trialeurodes vaporariorum, Dialeurodes spp., Aleurothrixus spp., Bemisia spp., Aleyrodes spp., ausserdem Trips-Arten und Wanzen.
– Acarina, wie z. B. Tetranychus urticae, Panonychus ulmi und andere Tetranychiden, Tarsonemiden wie Steneotarsonemus spp., Tenuipalpiden wie Brevipalpus, Eriophyiden wie Phyllocoptruta oleivora, Aceria sheldoni, Eriophyes spp., Aceria spp., ferner Zecken.
– Nematoda, wie z. B. freilebende Nematoden (u. a. Pratylenchus spp., wie P. penetrans), blattparasitische Nematoden (u. a. Aphelenchoides), wurzelparasitische Nematoden (u. a. Meloidogynae spp,. wie M. incognita, Globodera spp., wie G. rostochiensis).

Die erfindungsgemässen Wirkstoffe können in die üblichen Formulierungen übergeführt werden, wie Lösungen, Emulsionen, Suspensionen, Pulver, Pasten und Granulate. Diese werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln, und/oder Dispergiermitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten wie Xylol, Toluol, Benzol und Alkylnaphthaline; chlorierte Aromaten und chlorierte aliphatische Kohlenwasserstoffe wie Chlorbenzole, Chloräthylene und Methylenchlorid; aliphatische Kohlenwasserstoffe wie Cyclohexan und Paraffine, z. B. Erdölfraktionen; Alkohole, wie Butanol und Glykol, sowie deren Äther und Ester; Ketone wie Aceton, Methyläthylketon, Methylisobutylketon und Cyclohexanon; stark polare Lösungsmittel wie Dimethylformamid und Dimethylsulfoxid sowie Wasser; unter verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe, z. B. Freon; als feste Trägerstoffe: natürliche Gesteinsmehle wie Kaoline, Tonerden, Talkum, Kreide, Quarz, Attapulgit, Montmorillonit und Diatomeenerde und synthetische Gesteinsmehle wie hochdisperse Kieselsäure, Aluminiumoxid und Silikate; als Emulgiermittel: nichtionogene und anionische Emulgatoren wie Polyoxyäthylen-Fettsäure-Ester, Polyoxyäthylen-Fettalkohol-Äther, z. B. Alkylarylpolyglykoläther, Alkylsulfonate, Alkylsulfate und Arylsulfonate; als Dispergiermittel: z. B. Lignin, Sulfitablaugen und Methylcellulose.

Die erfindungsgemässen Wirkstoffe können in Formulierungen in Mischung mit anderen bekannten Wirkstoffen vorliegen. Die Formulierungen enthalten im allgemeinen zwischen 0,1 und 95 Gew.-% Wirkstoff, vorzugsweise zwischen 0,5 und 40 Gew.-%.

Die Wirkstoffe können als solche in Form ihrer Formulierungen oder in den daraus bereiteten Anwendungsformen wie gebrauchsfertige Lösungen, Emulsionen, Schäume, Suspensionen, Pulver, Pasten, lösliche Pulver, Stäubmittel und Granulate angewendet werden. Die Anwendung geschieht in üblicher Weise, z. B. durch Verspritzen, Versprühen, Vernebeln, Verstäuben, Verstreuen, Eindrillen, Verräuchern, Giessen, Beizen oder Inkrustieren.

Die Wirkstoffkonzentrationen in den anwendungsfertigen Zubereitungen können in grösseren Bereichen variiert werden. Im allgemeinen liegen sie zwischen 0,0001 und 10 Gew.-%, vorzugsweise zwischen 0,01 und 1 Gew.-%.

Die Wirkstoffe können auch mit gutem Erfolg im Ultra-Low-Volume-Verfahren (ULV) verwendet werden, wo es möglich ist, Formulierungen bis zu 95% oder sogar den 100%igen Wirkstoff allein auszubringen.

Die gute Wirkung gegen pflanzenschädigende Insekten, Nemathoden und Milben geht aus den nachstehenden Beispielen mit Blattläusen, Zikaden, Spinnmilben und weiteren Schädlingen hervor.

In den nachstehenden Beispielen ist die Verbindung mit der Formel

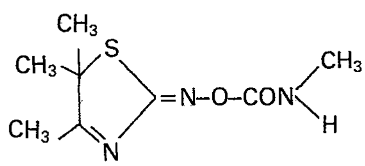

als Verbindung A bezeichnet.

Beispiel 1

Testinsekt: Leptinotarsa decemlineata, Kartoffelkäfer

Testmethode: Larvizid-Test auf Blattrondellen

Kartoffelblattrondellen werden mit acetonischer Wirkstofflösung besprüht. Pro Rondelle werden 5 frischgeschlüpfte Larven des ersten Stadiums angesetzt, bei 25°C und 60% relativer Feuchtigkeit inkubiert und nach 2 Tagen mit 2 unbehandelten Rondellen pro Parzelle nachgefüttert. Unbehandelte und mit Aceton behandelte Blattrondellen dienen als Kontrollen. Testdauer: 4 Tage. Die Resultate werden ausgedrückt als prozentuale Reduktion der Überlebensrate der Larven im Vergleich zu den Kontrollparzellen.

| Wirkstoff | Dosis ($10^{-x}$g AS/cm$^2$) | % Wirkung |
|---|---|---|
| Verbindung A | x = 5 | 100 |
| | 6 | 100 |
| | 7 | 100 |
| | 8 | 0 |

(Unbehandelte Kontrollparzellen: 5% Mortalität)
AS = Wirkstoff

Beispiel 2

Testinsekt: Tetranychus urticae, gemeine Spinnmilbe

Testmethode: Ovizid-Test auf Blattrondellen

Buschbohnen-Blattrondellen werden mit 10 adulten Weibchen infiziert. 1 Tag später werden die Weibchen entfernt, und die Blattrondellen mit 70 bis 100 Eiern werden mit acetonischer Wirkstofflösung besprüht. Die behandelten Rondellen werden auf feuchten Schaumstoff gelegt und bei 25°C und 60% relativer Feuchtigkeit inkubiert. Unbehandelte und mit Aceton behandelte Blattrondellen dienen als Kontrollen. Testdauer: 6 Tage. Die Resultate werden ausgedrückt als prozentuale Reduktion der Schlüpfrate der Eier.

| Wirkstoff | Dosis ($10^{-x}$g AS/cm$^2$) | % Wirkung |
|---|---|---|
| Verbindung A | x = 5 | 100 |
| | 6 | 100 |
| | 7 | 44 |

(Unbehandelte Kontrollparzellen: 3% Eimortalität)
AS = Wirkstoff

Beispiel 3

Testinsekt: Musca domestica, Stubenfliege

Testmethode: UV-Persistenztest auf Glas (die Stärke der Wirkstoffe wird neben der Wirkung nach 72 h Vorinkubation unter UV, auch durch den Vergleich mit der Variante ohne Vorinkubation erkannt.)

Petrischalenböden (∅ 9 cm) werden mit acetonischer Wirkstofflösung behandelt und während 72 h unter UV-Licht (Philips TOA 40 W/05) bei 28°C und 60% relativer Feuchtigkeit vorinkubiert. Nach dieser Zeit werden pro Petrischale 10 Fliegenweibchen (4–5 Tage alt) angesetzt und bei 25°C und 60% relative Feuchtigkeit inkubiert. Unbehandelte und mit Aceton behandelte Petrischalen dienen als Kontrollen. Testdauer: 72 h (unter UV) und 24 h (nach Ansatz der Fliegen). Die Resultate werden ausgedrückt als prozentuale Reduktion der Überlebensrate der Fliegen im Vergleich zu den Kontrollparzellen.

| Wirkstoff | Dosis ($10^{-x}$g AS/cm$^2$ | % Wirkung (nach 72 Std. Vorinkubation unter UV) | % Wirkung (ohne Vorinkubation) |
|---|---|---|---|
| Verbindung A | x = 5 | 100 | 100 |
| | 6 | 100 | 100 |
| | 7 | 0 | 95 |
| | 8 | | 2 |

(Unbehandelte Kontrollparzellen: 0% Mortalität)
AS = Wirkstoff

Erfindungsgemässe Verbindungen der Formel I und deren Herstellungsverfahren werden in den nachstehenden Beispielen 4 bis 25 illustriert.

Beispiel 4 (Verfahrensvariante a)

15,8 g (0,1 Mol) 2-Oxo-4,5,5-trimethyl-3-thiazolinoxim und 8,5 g (0,15 Mol) Methylisocyanat werden in 100 ml wasserfreiem Dichlormethan gelöst. Zu dieser Lösung gibt man einige Tropfen Triäthylamin hinzu. Das Gemisch erwärmt sich nach kurzer Zeit auf 40–45°C, eventuell wird kurze Zeit auf 50–60°C erwärmt. Anschliessend wird das Lösungsmittel im Vakuum abdestilliert. Der Rückstand wird in 50 ml heissem Essigsäureäthylester gelöst, mit Kohle behandelt, filtriert und das heisse Filtrat auf 20–30 ml eingeengt und mit 20–30 ml Hexan versetzt. Die ausgefallenen Kristalle werden abgenutscht, mit Äther gewaschen und getrocknet. Man erhält 2-Oxo-4,5,5-trimethyl-3-thiazolin-O-(methylcarbamoyl)oxim. Fp.: 123–125° C.

Beispiel 5 (Verfahrensvariante b)

15,8 g (0,1 Mol) 2-Oxo-4,5,5-trimethyl-3-thiazolinoxim und 15,2 g (0,15 Mol) Triäthylamin werden in 100 ml wasserfreiem 1,2-Dimethoxyäthan gelöst und 16,1 g (0,15 Mol) Dimethylcarbamoylchlorid zugetropft. Das Gemisch wird 3 h am Rückfluss erhitzt und anschliessend das Lösungsmittel im Vakuum abdestilliert. Der Rückstand wird in 100 ml Dichlormethan gelöst, mit Wasser gewaschen, die organische Phase über Natriumsulfat getrocknet und das Lösungsmittel abdestilliert. Der Rückstand wird chromatographisch an Kieselgel (Laufmittel: Äther/Dichlormethan 9:1) gereinigt. Man erhält 2-Oxo-4,5,5-trimethyl-3-thiazolin-O-(dimethylcarbamoyl)oxim. Fp.: 108–110°C.

Beispiel 6

Analog dem in Beispiel 4 beschriebenen Verfahren wird 2-Oxo-4-äthyl-5,5-dimethyl-3-thiazolinoxim mit Methylisocyanat umgesetzt. Man erhält 2-Oxo-4-äthyl-5,5-dimethyl-3-thiazolin-O-(methylcarbamoyl)oxim, Fp.: 98–100°C. Ausgangsmaterial: 2-Methyl-2-thiocyanat-3-pentanon. $Kp_{0,03}$: 56–58°C. 2-Oxo-4-äthyl-5,5-dimethyl-3-thiazolinoxim. Fp.: 178–180°C.

Beispiel 7

Analog dem in Beispiel 4 beschriebenen Verfahren wird 2-Oxo-4-isopropyl-5,5-dimethyl-3-thiazolin-oxim mit Methylisocyanat umgesetzt. Man erhält 2-Oxo-4-isopropyl-5,5-dimethyl-3-thiazolin-O-(methylcarbamoyl)oxim. Fp.: 89–91°C. Ausgangsmaterial: 2,4-Dimethyl-2-thiocyanat-3-pentanon. $Kp_{0,02}$: 64–66°C. 2-Oxo-4-isopropyl-5,5-dimethyl-3-thiazolin-oxim. Fp.: 220–222°C.

Beispiel 8

Analog dem in Beispiel 4 beschriebenen Verfahren wird 2-Oxo-4,5,5-trimethyl-3-thiazolin-oxim mit Äthylisocyanat umgesetzt. Man erhält 2-Oxo-4,5,5-trimethyl-3-thiazolin-O-(äthylcarbamoyl)-oxim. Fp.: 84–86°C.

Beispiel 9

Analog dem in Beispiel 4 beschriebenen Verfahren wird 2-Oxo-4-phenyl-5,5-dimethyl-3-thiazolinoxim mit Methylisocyanat umgesetzt. Man erhält 2-Oxo-4-phenyl-5,5-dimethyl-3-thiazolin-O-(methylcarbamoyl)oxim. Fp.: 135–137°C. Ausgangsmaterial: 2-Methyl-1-phenyl-2-thiocyanat-1-propanon. $Kp_{0,02}$: 98–100°C. 2-Oxo-4-phenyl-5,5-dimethyl-3-thiazolin-oxim. Fp.: 147–149°C.

Beispiel 10

Analog dem in Beispiel 4 beschriebenen Verfahren wird 2-Oxo-4,5,5-trimethyl-3-thiazolin-oxim mit Isopropylisocyanat umgesetzt. Man erhält 2-Oxo-4,5,5-trimethyl-3-thiazolin-O-(isopropylcarbamoyl)-oxim. Fp.: 114–116°C.

Beispiel 11

Analog dem in Beispiel 4 beschriebenen Verfahren wird 2-Oxo-4,5,5-trimethyl-3-thiazolin-oxim mit Cyclopropylisocyanat umgesetzt. Man erhält 2-Oxo-4,5,5-trimethyl-3-thiazolin-O-(cyclopropylcarbamoyl)-oxim. Fp.: 86–89°C.

Beispiel 12

Analog dem in Beispiel 4 beschriebenen Verfahren wird 2-Oxo-4,5,5-trimethyl-3-thiazolin-oxim mit Cyclobutylisocyanat umgesetzt. Man erhält 2-Oxo-4,5,5-trimethyl-3-thiazolin-O-(cyclobutylcarbamoyl)-oxim. Fp.: 94–96°C.

Beispiel 13

Analog dem in Beispiel 4 beschriebenen Verfahren wird 2-Oxo-4-propyl-5,5-dimethyl-3-thiazolinoxim mit Methylisocyanat umgesetzt. Man erhält 2-Oxo-4-propyl-5,5-dimethyl-3-thiazolin-O-(methylcarbamoyl)-oxim. Fp.: 112–114°C. Ausgangsmaterial: 2-Oxo-4-propyl-5,5-dimethyl-3-thiazolinoxim. Fp.: 145–147°C.

Beispiel 14

Analog dem in Beispiel 4 beschriebenen Verfahren wird 2-Oxo-4-cyclohexyl-5,5-dimethyl-3-thiazolin-oxim mit Methylisocyanat umgesetzt. Man erhält 2-Oxo-4-cyclohexyl-5,5-dimethyl-3-thiazolin-O-(methylcarbamoyl)-oxim. Fp.: 158–160°C. Ausgangsmaterial: 2-Oxo-4-cyclohexyl-5,5-dimethyl-3-thiazolin-oxim. Fp.: 159–162°C.

Beispiel 15

Analog dem in Beispiel 4 beschriebenen Verfahren wird 2-Oxo-4,5-dimethyl-5-äthyl-3-thiazolinoxim mit Methylisocyanat umgesetzt. Man erhält 2-Oxo-4,5-dimethyl-5-äthyl-3-thiazolin-O-(methylcarbamoyl)-oxim. Fp.: 115–117°C. Ausgangsmaterial: 2-Oxo-4,5-dimethyl-5-äthyl-3-thiazolinoxim. Fp.: 143,5–144,5°C.

Beispiel 16

Analog dem in Beispiel 4 beschriebenen Verfahren wird 2-Oxo-4,5-diäthyl-5-methyl-3-thiazolinoxim mit Methylisocyanat umgesetzt. Man erhält 2-Oxo-4,5-diäthyl-5-methyl-3-thiazolin-O-(methylcarbamoyl)-oxim. Fp. 88–90°C. Ausgangsmaterial: 2-Oxo-4,5-diäthyl-5-methyl-3-thiazolin-oxim. Fp.: 125–128°C.

Beispiel 17

Analog dem in Beispiel 4 beschriebenen Verfahren wird 2-Oxo-4,5-dimethyl-5-propyl-3-thiazolinoxim mit Methylisocyanat umgesetzt. Man erhält 2-Oxo-4,5-dimethyl-5-propyl-3-thiazolin-O-(methylcarbamoyl)-oxim. Fp.: 66–69°C. Ausgangsmaterial: 2-Oxo-4,5-dimethyl-5-propyl-3-thiazolinoxim. Fp.: 94–96°C.

Beispiel 18

Analog dem in Beispiel 4 beschriebenen Verfahren wird 2-Oxo-4-propyl-5-äthyl-5-methyl-3-thiazolin-oxim mit Methylisocyanat umgesetzt. Man erhält 2-Oxo-4-propyl-5-äthyl-5-methyl-3-thiazolin-O-(methylcarbamoyl)-oxim. Fp. 124–126°C. Ausgangsmaterial: 2-Oxo-4-propyl-5-äthyl-5-methyl-3-thiazolin-oxim. Fp. 132–135°C.

Beispiel 19

Analog dem in Beispiel 4 beschriebenen Verfahren wird 2-Oxo-4-butyl-5,5-dimethyl-3-thiazolin-oxim mit Methylisocyanat umgesetzt. Man erhält 2-Oxo-4-butyl-5,5-dimethyl-3-thiazolin-O-(methylcarbamoyl)-oxim. Fp.: 128–130°C. Ausgangsmaterial: 2-Oxo-4-butyl-5,5-dimethyl-3-thiazolin-oxim- Fp.: 117–119°C.

Beispiel 20

Analog dem in Beispiel 4 beschriebenen Verfahren wird 2-Oxo-4,5-dimethyl-5-isobutyl-3-thiazolin-oxim mit Methylisocyanat umgesetzt. Man erhält 2-Oxo-4,5-dimethyl-5-isobutyl-3-thiazolin-O-(methylcarbamoyl)-oxim. Fp.: 99–101°C. Ausgangsmaterial: 2-Oxo-4,5-dimethyl-5-isobutyl-3-thiazolin-oxim. Fp.: 105–107°C.

Beispiel 21

Analog dem in Beispiel 4 beschriebenen Verfahren wird 2-Oxo-4,5-dimethyl-5-pentyl-3-thiazolin-oxim mit Methylisocyanat umgesetzt. Man erhält 2-Oxo-4,5-dimethyl-5-pentyl-3-thiazolin-O-(methylcarbamoyl)-oxim als dickflüssiges Öl. $n_D^{44,5}$: 1,5256. Ausgangsmaterial: 2-Oxo-4,5-dimethyl-5-pentyl-3-thiazolin-oxim. Fp.: 104–106°C.

Beispiel 22

Analog dem in Beispiel 4 beschriebenen Verfahren wird 2-Oxo-4-(p-chlorphenyl)-5,5-dimethyl-3-thiazolin-oxim mit Methylisocyanat umgesetzt. Man erhält 2-Oxo-4-(p-chlorphenyl)-5,5-dimethyl-3-thiazolin-O-(methylcarbamoyl)-oxim. Fp.: 147–149°C. Ausgangsmaterial: 2-Oxo-4-(p-chlorphenyl)-5,5-dimethyl-3-thiazolin-oxim. Fp.: 205–207° C.

Beispiel 23

Analog dem in Beispiel 4 beschrieben Verfahren wird 2-Oxo-4,5-dimethyl-5-butyl-3-thiazolin-oxim mit Methylisocyanat umgesetzt. Man erhält 2-Oxo-4,5-dimethyl-5-butyl-3-thiazolin-O-(methylcarbamoyl)-oxim als dickflüssiges Öl. $n_D^{44,5}$: 1,5324. Ausgangsmaterial: 2-Oxo-4,5-dimethyl-5-butyl-3-thiazolin-oxim. Fp.: 100–102°C.

Beispiel 24

Analog dem in Beispiel 4 beschriebenen Verfahren wird 2-Oxo-4,5-dimethyl-5-(3-methyl-2-pentenyl)-3-thiazolin-oxim mit Methylisocyanat umgesetzt. Man erhält 2-Oxo-4,5-dimethyl-5-(3-methyl-2-pentenyl)-3-thiazolin-O-(methylcarbamoyl)-oxim als dickflüssiges Öl; $n_D^{20}$ : 1,5424.

Das als Ausgangsmaterial benötigte 3-Brom-3,6-dimethyl-5-octen-2-on kann wie folgt hergestellt werden:

5 g 3,6-Dimethyl-5-octen-2-on werden vorgelegt und tropfenweise mit einer Lösung von 18,5 g 2-Carboxyäthyltriphenylphosphoniumperbromid in 30 ml Tetrahydrofuran versetzt. Anschliessend lässt man über Nacht bei Raumtemperatur rühren. Das unlösliche Bromid wird abfiltriert, das Filtrat wird auf 5% wässrige Natriumcarbonatlösung gegossen und zweimal mit Äther extrahiert. Die Extrakte werden mit Wasser und Kochsalzlösung gewaschen, über Natriumsulfat getrocknet und eingedampft. Der Rückstand wird im Kugelrohr bei 60°C/0,06 Torr destilliert. Man erhält rohes 3-Brom-3,6-dimethyl-5-octen-2-on, das gemäss dem unten in Beispiel 26 beschriebenen Verfahren zum Rhodanid und weiter zum 2-Oxo-4,5-dimethyl-5-(3-methyl-2-pentenyl)-3-thiazolin-oxim, $n_D^{20}$ : 1,5432, umgesetzt wird.

Beispiel 25

7,9 g 2-Oxo-4,5,5-trimethyl-3-thiazolin-oxim werden in 100 ml Acetonitril gelöst. Zu dieser Lösung gibt man 6,9 g Kaliumcarbonat hinzu. Das Reaktionsgemisch wird auf 0°C gekühlt und unter intensivem Rühren tropfenweise mit einer Lösung von 6,8 g N-Acetyl-N-methyl-carbamidsäurechlorid in 25 ml Acetonitril versetzt. Nach erfolgter Zugabe wird das Eisbad entfernt und während 2 h bei Raumtemperatur gerührt. Zur Aufarbeitung wird das Reaktionsgemisch filtriert und das Filtrat am Rotationsverdampfer eingeengt. Der Rückstand wird auf halbgesättigte Kochsalzlösung gegossen und dreimal mit Essigester extrahiert. Die Extrakte werden mit halbgesättigter und gesättigter Kochsalzlösung gewaschen, über Natriumsulfat getrocknet und eingedampft. Durch Kristallisation aus Hexan-Essigester erhält man reines 2-Oxo-4,5,5-trimethyl-3-thiazolin-O-[methyl(acetyl)-carbamoyl]-oxim. Fp.: 119–124°C.

Analog dem oben beschriebenen Verfahren wird 2-Oxo-4,5-dimethyl-5-äthyl-3-thiazolin-oxim mit N-Acetyl-N-methylcarbamidsäurechlorid umgesetzt. Man erhält 2-Oxo-4,5-dimethyl-5-äthyl-3-thiazolin-O-[methyl(acetyl)carbamoyl]oxim; Fp.: 98–101°C.

Beispiel 26

Das als Ausgangsmaterial in den Beispielen 4, 5, 8, 10 bis 12 und 25 benötigte 2-Oxo-4,5,5-trimethyl-3-thiazolin-oxim kann wie folgt hergestellt werden:

16,5 g (0,1 Mol) 3-Brom-3-methyl-2-butanon und 11,6 g (0,12 Mol) Kaliumrhodanid werden in 150 ml wasserfreiem Aceton 1–2 h am Rückfluss erhitzt. Nach dem Abkühlen wird das ausgeschiedene Kaliumbromid abgenutscht, mit etwas Aceton nachgewaschen und im Vakuum bei 50°C zur Trockene eingedampft. Der Rückstand wird in 200 ml Äther aufgenommen, dreimal mit 100 ml Wasser gewaschen, die Ätherphase über Natriumsulfat getrocknet und im Vakuum zur Trockene eingedampft. Das Rohprodukt wird an einer kurzen Vigreux-Kolonne fraktioniert. Man erhält 3-Methyl-3-thiocyanat-2-butanon. $Kp_{0,04}$: 52°C.

8,3 g (0,12 Mol) Hydroxylaminhydrochlorid werden in 50 ml Wasser gelöst und mit 10,1 g (0,12

Mol) Natriumhydrogencarbonat versetzt. Diese Lösung (pH 6–7) wird bei Raumtemperatur unter Rühren zu einer Lösung aus 14,3 g (0,1 Mol) 3-Methyl-3-thiocyanat-2-butanon in 100 ml Äthanol hinzugefügt. Die Temperatur des Reaktionsgemisches steigt rasch auf 37°C an. Anschliessend wird 1 h nachgerührt und 120 ml des Lösungsmittels im Vakuum bei 50°C abdestilliert. Der Rückstand wird dreimal mit je 100 ml Äther ausgeschüttelt, die organische Phase mit wenig Wasser gewaschen, über Natriumsulfat getrocknet und bis zur beginnenden Kristallisation eingeengt. Nach dem Abkühlen wird der Kristallbrei mit 10 ml Hexan versetzt, die Kristalle abgenutscht, mit Äther/Hexan 2:1 gewaschen und getrocknet. Man erhält 2-Oxo-4,5,5-trimethyl-3-thiazolin-oxim. Fp.: 151–153°C.

Die entsprechenden Ausgangsmaterialien in den Beispielen 6, 7, 9 und 13 bis 24 werden in Analogie zum vorstehenden Beispiel hergestellt.

Beispiel 27

Die Verbindungen der Formel I können beispielsweise wie folgt formuliert werden:

| | | g/Liter |
|---|---|---|
| a) | Wirkstoff der Formel I | 250 |
| | N-Methyl-2-pyrrolidon | 500 |
| | Emulgator-Mischung bestehend aus: Calcium-Alkylarylsulfonat, Alkylphenoläthoxylat, Blockpolymerisat aus Propylenoxid und Äthylenoxid | 50 |
| | Calcium-Dodecylbenzolsulfonat | 25 |
| | Lösungsmittel (Gemisch aus Mono-, Di- und Tri-nieder Alkylbenzolen) | ad 1000 ml |

| | | g/Liter |
|---|---|---|
| b) | Wirkstoff der Formel I | 250 |
| | N-Methyl-2-pyrrolidon | ad 1000 ml |

| | | |
|---|---|---|
| c) | Wirkstoff der Formel I | 50 g |
| | Hochdisperse Kieselsäure | 5 g |
| | Natrium-Laurylsulfat | 1 g |
| | Natrium-Lignonsulfonat | 2 g |
| | Kaolin | 42 g |
| | | 100 g |

**Patentansprüche für die Vertragsstaaten: BE, CH, DE, FR, GB, IT, LU, NL, SE**

1. Verbindungen der allgemeinen Formel

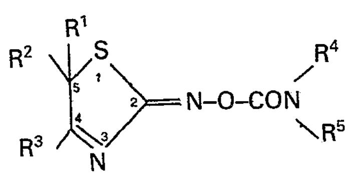

I

worin $R^1$ und $R^3$ $C_{1-5}$-Alkyl, $C_{2-5}$-Alkenyl, $C_{3-6}$-Cycloalkyl oder Phenyl oder Naphthyl bedeuten, wobei der Phenyl- oder Naphthylrest gegebenenfalls mit einem Halogenatom substituiert sein kann, $R^2$ $C_{1-5}$-Alkyl, $R^4$ $C_{1-5}$-Alkyl oder $C_{3-6}$-Cycloalkyl und $R^5$ Wasserstoff, $C_{1-5}$-Alkyl, $C_{2-6}$-Alkylcarbonyl oder mit Halogen substituiertes $C_{2-6}$-Alkylcarbonyl bedeuten, und Säureadditionssalze dieser Verbindungen.

2. Verbindungen nach Anspruch 1, worin $R^1$, $R^2$ und $R^3$ Alkyl mit 1–3 Kohlenstoffatomen bedeuten.

3. Verbindungen nach Anspruch 1 oder Anspruch 2, worin $R^4$ Alkyl mit 1–3 Kohlenstoffatomen und $R^5$ Wasserstoff bedeuten.

4. 2-Oxo-4,5,5-trimethyl-3-thiazolin-O-(methylcarbamoyl)-oxim und dessen Säureadditionssalze.

5. 2-Oxo-4,5-dimethyl-5-äthyl-3-thiazolin-O-(methylcarbamoyl)-oxim und dessen Säureadditionssalze.

6. 2-Oxo-4,5-diäthyl-5-methyl-3-thiazolin-O-(methylcarbamoyl)-oxim und dessen Säureadditionssalze.

7. Eine Verbindung der Formel

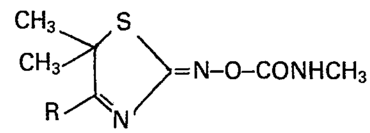

Ia

worin R Äthyl oder Isopropyl ist.

8. Eine Verbindung der Formel

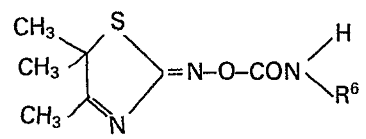

Ib

worin $R^6$ Äthyl, Isopropyl, Cyclopropyl oder Cyclobutyl ist.

9. Verbindungen nach einem der Ansprüche 1 bis 8 zur Verwendung bei der Bekämpfung von Insekten.

10. Schädlingsbekämpfungsmittel, dadurch gekennzeichnet, dass es eine wirksame Menge von mindestens einer Verbindung der allgemeinen Formel I in Anspruch 1 oder einem Säureadditionssalz einer solchen Verbindung sowie inertes Trägermaterial enthält.

11. Schädlingsbekämpfungsmittel, dadurch gekennzeichnet, dass es eine wirksame Menge von 2-Oxo-4,5,5-trimethyl-3-thiazolin-O-(methylcarbamoyl)-oxim oder einem Säureadditionssalz davon sowie inertes Trägermaterial enthält.

12. Schädlingsbekämpfungsmittel, dadurch gekennzeichnet, dass es eine wirksame Menge von 2-Oxo-4,5-dimethyl-5-äthyl-3-thiazolin-O-(methylcarbamoyl)-oxim oder einem Säureadditionssalz davon sowie inertes Trägermaterial enthält.

13. Schädlingsbekämpfungsmittel, dadurch gekennzeichnet, dass es eine wirksame Menge von 2-Oxo-4,5-diäthyl-5-methyl-3-thiazolin-O-(methylcarbamoyl)-oxim oder einem Säureadditionssalz davon sowie inertes Trägermaterial enthält.

14. Verfahren zur Herstellung der Verbindungen der Formel I und deren Salze gemäss Anspruch 1, dadurch gekennzeichnet, dass man ein Oxim der Formel

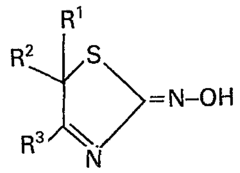 II

worin $R^1$, $R^2$ und $R^3$ die im Anspruch 1 angegebene Bedeutung besitzen,

a) mit einem Isocyanat der Formel

$$R^4{-}N{=}C{=}O \quad III$$

in welcher $R^4$ die im Anspruch 1 angegebene Bedeutung hat, in Gegenwart eines Verdünnungsmittels und gegebenenfalls in Gegenwart eines Katalysators umsetzt, oder

b) mit einem Carbamoylhalogenid der Formel

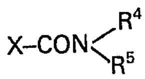 IV

in welcher $R^4$ und $R^5$ die im Anspruch 1 angegebene Bedeutung haben und X ein Halogenatom bedeutet,
in Gegenwart eines Verdünnungsmittels und eines säurebindenden Mittels umsetzt, und erwünschtenfalls eine erhaltene Verbindung der Formel I mit einer Säure in ein Säureadditionssalz überführt.

15. Verwendung einer in den Ansprüchen 1 bis 8 genannten Verbindung bzw. eines in den Ansprüchen 10 bis 13 genannten Mittels zur Bekämpfung von Schädlingen.

**Patentansprüche für den Vertragsstaat: AT**

1.Schädlingsbekämpfungsmittel, dadurch gekennzeichnet, dass es eine wirksame Menge von mindestens einer Verbindung der allgemeinen Formel

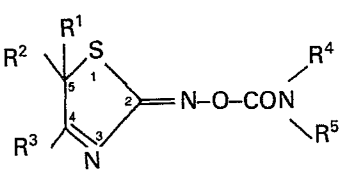 I

worin $R^1$ und $R^3$ $C_{1-5}$-Alkyl, $C_{2-5}$-Alkenyl, $C_{3-6}$-Cycloalkyl oder Phenyl oder Naphthyl bedeuten, wobei der Phenyl- oder Naphthylrest gegebenenfalls mit einem Halogenatom substituiert sein kann, $R^2$ $C_{1-5}$-Alkyl, $R^4$ $C_{1-5}$-Alkyl oder $C_{3-6}$-Cycloalkyl und $R^5$ Wasserstoff, $C_{1-5}$-Alkyl, $C_{2-6}$-Alkylcarbonyl oder mit Halogen substituiertes $C_{2-6}$-Alkylcarbonyl bedeuten, oder einem Säureadditionssalz einer solchen Verbindung, sowie inertes Trägermaterial enthält.

2. Schädlingsbekämpfungsmittel nach Anspruch 1, dadurch gekennzeichnet, dass es eine wirksame Menge von mindestens einer Verbindung der allgemeinen Formel I, worin $R^1$, $R^2$ und $R^3$ Alkyl mit 1–3 Kohlenstoffatomen bedeuten, oder einem Säureadditionssalz einer solchen Verbindung, sowie inertes Trägermaterial enthält.

3. Schädlingsbekämpfungsmittel nach Anspruch 1 oder Anspruch 2, dadurch gekennzeichnet, dass es eine wirksame Menge von mindestens einer Verbindung der allgemeinen Formel I, worin $R^4$ Alkyl mit 1–3 Kohlenstoffatomen und $R^5$ Wasserstoff bedeuten, oder einem Säureadditionssalz einer solchen Verbindung, sowie inertes Trägermaterial enthält.

4. Schädlingsbekämpfungsmittel nach Anspruch 1, dadurch gekennzeichnet, dass es eine wirksame Menge von 2-Oxo-4,5,5-trimethyl-3-thiazolin-O-(methylcarbamoyl)-oxim, oder einem Säureadditionssalz davon, sowie inertes Trägermaterial enthält.

5. Schädlingsbekämpfungsmittel nach Anspruch 1, dadurch gekennzeichnet, dass es eine wirksame Menge von 2-Oxo-4,5-dimethyl-5-äthyl-3-thiazolin-O-(methylcarbamoyl)-oxim, oder einem Säureadditionssalz davon, sowie inertes Trägermaterial enthält.

6. Schädlingsbekämpfungsmittel nach Anspruch 1, dadurch gekennzeichnet, dass es eine wirksame Menge von 2-Oxo-4,5-diäthyl-5-methyl-3-thiazolin-O-(methylcarbamoyl)-oxim, oder einem Säureadditionssalz davon, sowie inertes Trägermaterial enthält.

7. Schädlingsbekämpfungsmittel nach Anspruch 1, dadurch gekennzeichnet, dass es eine wirksame Menge einer Verbindung der Formel

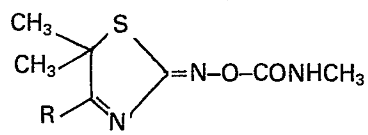 Ia

worin R Äthyl oder Isopropyl ist, enthält.

8. Schädlingsbekämpfungsmittel nach Anspruch 1, dadurch gekennzeichnet, dass es eine wirkame Menge einer Verbindung der Formel

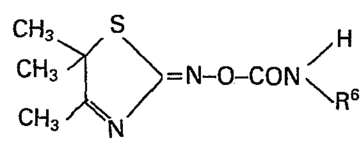 Ib

worin $R^6$ Äthyl, Isopropyl, Cyclopropyl oder Cyclobutyl ist, enthält.

9. Verfahren zur Herstellung der Verbindungen der Formel I in Anspruch 1, und deren Salze, dadurch gekennzeichnet, dass man ein Oxim der Formel

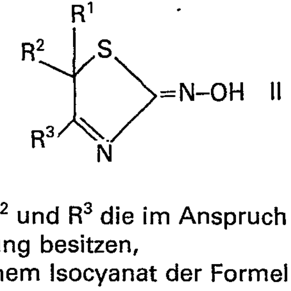 II

worin $R^1$, $R^2$ und $R^3$ die im Anspruch 1 angegebene Bedeutung besitzen,

a) mit einem Isocyanat der Formel

$$R^4{-}N{=}C{=}O \quad III$$

in welcher $R^4$ die im Anspruch 1 angegebene Bedeutung hat, in Gegenwart eines Verdünnungsmittels und gegebenenfalls in Gegenwart eines Katalysators umsetzt, oder

b) mit einem Carbamoylhalogenid der Formel

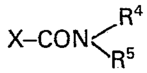 IV

in welcher $R^4$ und $R^5$ die im Anspruch 1 angegebene Bedeutung haben und X ein Halogenatom bedeutet,
in Gegenwart eines Verdünnungsmittels und eines säurebindenden Mittels umsetzt, und erwünschtenfalls eine erhaltene Verbindung der Formel I mit einer Säure in ein Säureadditionssalz überführt.

10. Verwendung einer in den Ansprüchen 1 bis 8 genannten Verbindung zur Bekämpfung von Schädlingen.

**Claims for Contracting States: BE, CH, DE, FR, GB, IT, LU, NL, SE**

1. Compounds of the general formula

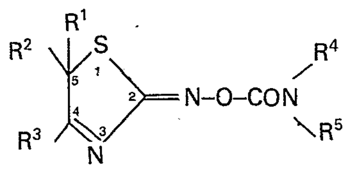 I

wherein $R^1$ and $R^3$ signify $C_{1-5}$-alkyl, $C_{2-5}$-alkenyl, $C_{3-6}$-cycloalkyl or phenyl or naphthyl, whereby the phenyl or naphthyl residue can be substituted, if desired, with a halogen atom, $R^2$ signifies $C_{1-5}$-alkyl, $R^4$ signifies $C_{1-5}$-alkyl or $C_{3-6}$-cycloalkyl and $R^5$ signifies hydrogen, $C_{1-5}$-alkyl, $C_{2-6}$-alkylcarbonyl or $C_{2-6}$-alkylcarbonyl substituted with halogen, and acid addition salts of these compounds.

2. Compounds according to claim 1, wherein $R^1$, $R^2$ and $R^3$ signify alkyl with 1–3 carbon atoms.

3. Compounds according to claim 1 or claim 2, wherein $R^4$ signifies alkyl with 1–3 carbon atoms and $R^5$ signifies hydrogen.

4. 2-Oxo-4,5,5-trimethyl-3-thiazoline-O-(methylcarbamoyl)-oxime and its acid addition salts.

5. 2-Oxo-4,5-dimethyl-5-ethyl-3-thiazoline-O-(methylcarbamoyl)-oxime and its acid addition salts.

6. 2-Oxo-4,5-diethyl-5-methyl-3-thiazoline-O-(methylcarbamoyl)-oxime and its acid addition salts.

7. A compound of the formula

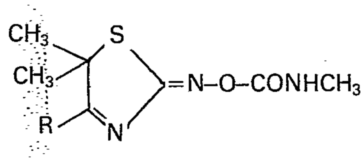 Ia

wherein R is ethyl or isopropyl.

8. A compound of the formula

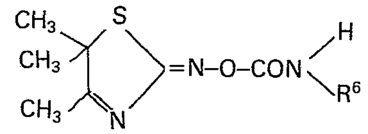 Ib

wherein $R^6$ is ethyl, isopropyl, cyclopropyl or cyclobutyl.

9. Compounds according to any one of claims 1 to 8 for use in the control of insects.

10. Pest control composition, characterized in that it contains an effective amount of at least one compound of general formula I in claim 1 or an acid addition salt of such a compound as well as inert carrier material.

11. Pest control composition, characterized in that it contains an effective amount of 2-oxo-4,5,5-trimethyl-3-thiazoline-O-(methylcarbamoyl)-oxime or an acid addition salt thereof as well as inert carrier material.

12. Pest control composition, characterized in that it contains an effective amount of 2-oxo-4,5-dimethyl-5-ethyl-3-thiazoline-O-(methylcarbamoyl)-oxime or an acid addition salt thereof as well as inert carrier material.

13. Pest control composition, characterized in that it contains an effective amount of 2-oxo-4,5-diethyl-5-methyl-3-thiazoline-O-(methylcarbamoyl)-oxime or an acid addition salt thereof as well as inert carrier material.

14. Process for the manufacture of the compounds of formula I and their salts in accordance with claim 1, characterized by reacting an oxime of the formula

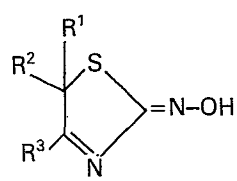 II

wherein $R^1$, $R^2$ and $R^3$ have the significance given in claim 1,

a) with an isocyanate of the formula

$R^4-N=C=O$ III

in which $R^4$ has the significance given in claim 1, in the presence of a diluent and, if desired, in the presence of a catalyst, or

b) with a carbamoyl halide of the formula

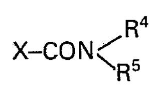 IV

in which $R^4$ and $R^5$ have the significance given in claim 1 and X signifies a halogen atom, in the presence of a diluent and an acid-binding agent, and, if desired, converting a compound of formula I obtained with an acid into an acid addition salt.

15. Use of a compound set forth in claims 1 to 8 or of a composition set forth in claims 10 to 13 for the control of pests.

**Claims for the contracting state: AT**

1. Pest control composition, characterized in that it contains an effective amount of at least one compound of the general formula

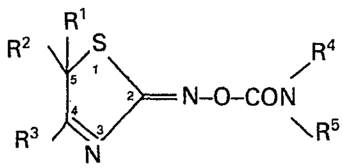

I

wherein $R^1$ and $R^3$ signify $C_{1-5}$-alkyl, $C_{2-5}$-alkenyl, $C_{3-6}$-cycloalkyl or phenyl or naphthyl, whereby the phenyl or naphthyl residue can be substituted, if desired, with a halogen atom, $R^2$ signifies $C_{1-5}$-alkyl, $R^4$ signifies $C_{1-5}$-alkyl or $C_{3-6}$-cycloalkyl and $R^5$ signifies hydrogen, $C_{1-5}$-alkyl, $C_{2-6}$-alkylcarbonyl or $C_{2-6}$-alkylcarbonyl substituted with halogen, or an acid addition salt of such a compound, as well as inert carrier material.

2. Pest control composition according to claim 1, characterized in that it contains an effective amount of at least one compound of general formula I, wherein $R^1$, $R^2$ and $R^3$ signify alkyl with 1–3 carbon atoms, or an acid addition salt of such a compound, as well as inert carrier material.

3. Pest control composition according to claim 1 or claim 2, characterized in that it contains an effective amount of at least one compound of general formula I, wherein $R^4$ signifies alkyl with 1–3 carbon atoms and $R^5$ signifies hydrogen, or an acid addition salt of such a compound, as well as inert carrier material.

4. Pest control composition according to claim 1, characterized in that it contains an effective amount of 2-oxo-4,5,5-trimethyl-3-thiazoline-O-(methylcarbamoyl)-oxime, or an acid addition salt thereof, as well as inert carriert material.

5. Pest control composition according to claim 1, characterized in that it contains an effective amount of 2-oxo-4,5-dimethyl-5-ethyl-3-thiazoline-O-(methylcarbamoyl)-oxime, or an acid addition salt thereof, as well as inert carrier material.

6. Pest control composition according to claim 1, characterized in that it contains an effective amount of 2-oxo-4,5-diethyl-5-methyl-3-thiazoline-O-(methylcarbamoyl)-oxime, or an acid addition salt thereof, as well as inert carrier material.

7. Pest control composition according to claim 1, characterized in that it contains an effective amount of a compound of the formula

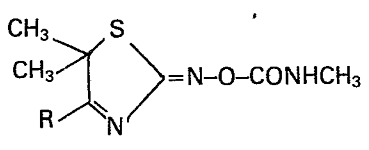

Ia

wherein R is ethyl or isopropyl.

8. Pest control composition according to claim 1, characterized in that it contains an effective amount of a compound of the formula

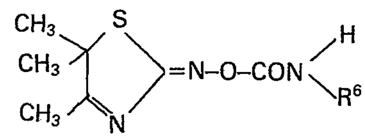

Ib

wherein $R^6$ is ethyl, isopropyl, cyclopropyl or cyclobutyl.

9. Process for the manufacture of the compounds of formula I in claim 1 and their salts, characterized by reacting an oxime of the formula

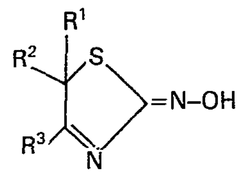

II

wherein $R^1$, $R^2$ and $R^3$ have the significance given in claim 1,

a) with an isocyanate of the formula

$$R^4\text{-}N{=}C{=}O \quad \text{III}$$

in which $R^4$ has the significance given in claim 1, in the presence of a diluent and, if desired, in the presence of a catalyst, or

b) with a carbamoyl halide of the formula

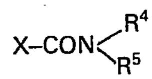

IV

in which $R^4$ and $R^5$ have the significance given in claim 1 and X signifies a halogen atom, in the presence of a diluent and an acid-binding agent, and, if desired, converting a compound of formula I obtained with an acid into an acid addition salt.

10. Use of a compound set forth in claims 1 to 8 for the control of pests.

**Revendications pour les états contractuels: BE, CH, DE, FR, GB, IT, LU, NL, SE**

1. Composés de formule générale

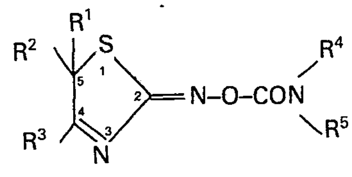

I

dans laquelle $R^1$ et $R^3$ représentent des groupes alkyles en $C_1-C_5$, alcényles en $C_2-C_5$, cycloalkyles en $C_3-C_6$ ou phényle ou naphtyle, les restes phényle ou naphtyle pouvant éventuellement être substitués par un atome d'halogène, $R^2$ représente un groupe alkyle en $C_1-C_5$, $R^4$ représente un groupe alkyle en $C_1-C_5$ ou cycloalkyle en $C_3-C_6$ et $R^5$ représente l'hydrogène, un groupe alkyle en $C_1-C_5$, alkylcarbonyle en $C_2-C_6$ ou alkylcarbonyle en $C_2-C_6$ substitué par un halogène, et les sels de

ces composés formés par addition avec des acides.

2. Composés selon la revendication 1, dans lesquels $R^1$, $R^2$ et $R^3$ représentent des groupes alkyles en $C_1-C_3$.

3. Composés selon la revendication 1 ou la revendication 2, dans lesquels $R^4$ représente un groupe alkyle en $C_1-C_3$ et $R^5$ représente l'hydrogène.

4. La 2-oxo-4,5,5-triméthyl-3-thiazoline-O-(méthylcarbamoyl)-oxime et ses sels formés par addition avec des acides.

5. La 2-oxo-4,5-diméthyl-5-éthyl-3-thiazoline-O-(méthylcarbamoyl)-oxime et ses sels formés par addition avec des acides.

6. La 2-oxo-4,5-diéthyl-5-méthyl-3-thiazoline-O-(méthylcarbamoyl)-oxime et ses sels formés par addition avec des acides.

7. Un composé de formule

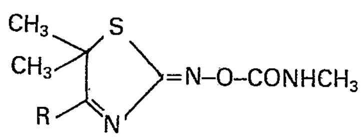 Ia

dans laquelle R représente un groupe éthyle ou isopropyle.

8. Un composé de formule

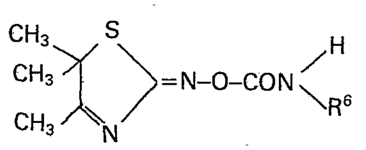 Ib

dans laquelle $R^6$ représente un groupe éthyle, isopropyle, cyclopropyle ou cyclobutyle.

9. Composés selon l'une des revendications 1 à 8, pour l'utilisation dans la lutte contre les insectes.

10. Produit pesticide caractérisé en ce qu'il contient une quantité efficace d'au moins un composé de formule générale I de la revendication 1 ou d'un sel d'un tel composé formé par addition avec un acide, et en véhicule inerte.

11. Produit pesticide caractérisé en ce qu'il contient une quantité efficace de 2-oxo-4,5,5-triméthyl-3-thiazoline-O-(méthylcarbamoyl)-oxime ou d'un sel de ce composé formé par addition avec un acide, et un véhicule inerte.

12. Produit pesticide caractérisé en ce qu'il contient une quantité efficace de 2-oxo-4,5-diméthyl-5-éthyl-3-thiazoline-O-(méthylcarbamoyl)-oxime ou d'un sel de ce composé formé par addition avec un acide, et un véhicule inerte.

13. Produit pesticide caractérisé en ce qu'il contient une quantité efficace de 2-oxo-4,5-diéthyl-5-méthyl-3-thiazoline-O-(méthylcarbamoyl)-oxime ou d'un sel de ce composé formé par addition avec un acide, et un véhicule inerte.

14. Procédé de préparation des composés de formule I et de leurs sels selon la revendication 1, caractérisé en ce que l'on fait réagir une oxime de formule

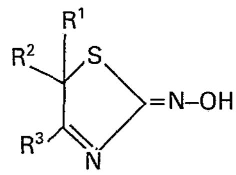 II

dans laquelle $R^1$, $R^2$ et $R^3$ ont les significations indiquées dans la revendication 1,

a) avec un isocyanate de formule

$$R^4{-}N{=}C{=}O \quad III$$

dans laquelle $R^4$ a la signification indiquée dans la revendication 1, en présence d'un diluant et le cas échéant en présence d'un catalyseur, ou bien

b) avec un halogénure de carbamoyle de formule

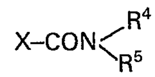 IV

dans laquelle $R^4$ et $R^5$ ont les significations indiquées dans la revendication 1 et X représente un atome d'halogène, en présence d'un diluant et d'un agent fixant les acides, et si on le désire, on convertit un composé obtenu de formule I, à l'aide d'un acide, en un sel formé par addition avec un acide.

15. Utilisation de l'un des composés mentionnés dans les revendications 1 à 8 ou de l'un des produits mentionnés dans les revendications 10 à 13 dans la lutte contre les parasites.

**Revendications pour l'état contractuel AT**

1. Produit pesticide caractérisé en ce qu'il contient une quantité efficace d'au moins un composé de formule générale

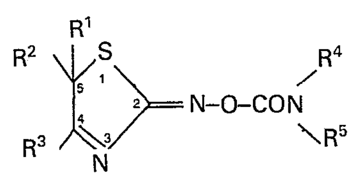 I

dans laquelle $R^1$ et $R^3$ représentent des groupes alkyles en $C_1-C_5$, alcényles en $C_2-C_5$, cycloalkyles en $C_3-C_6$ ou phényle ou naphthyle, les groupes phényle ou naphtyle pouvant éventuellement être substitués par un atome d'halogène, $R^2$ représente un groupe alkyle en $C_1-C_5$, $R^4$ représente un groupe alkyle en $C_1-C_5$ ou cycloalkyle en $C_3-C_6$ et $R^5$ représente l'hydrogène, un groupe alkyle en $C_1-C_5$, alkylcarbonyle, en $C_2-C_6$ ou alkylcarbonyle en $C_2-C_6$ substitué par un halogène, ou d'un sel d'un tel composé formé par addition avec un acide, et un véhicule inerte.

2. Produit pesticide selon la revendication 1, caractérisé en ce qu'il contient une quantité efficace d'au moins un composé de formule générale I dans laquelle $R^1$, $R^2$ et $R^3$ représentent des groupes alkyles en $C_1-C_3$, ou d'un sel d'un tel

composé formé par addition avec un acide, et un véhicule inerte.

3. Produit pesticide celon la revendication 1 ou 2, caractérisé en ce qu'il contient une quantité efficace d'au moins un composé de formule générale I dans laquelle $R^4$ représente un groupe alkyle en $C_1-C_3$ et $R^5$ représente l'hydrogène, ou d'un sel d'un tel composé formé par addition avec un acide, et un véhicule inerte.

4. Produit pesticide selon la revendication 1, caractérisé en ce qu'il contient une quantité efficace de 2-oxo-4,5,5-triméthyl-3-thiazoline-O-(méthylcarbamoyl)-oxime ou d'un sel de ce composé formé par addition avec un acide, et un véhicule inerte.

5. Produit pesticide selon la revendication 1, caractérisé en ce qu'il contient une quantité efficace de 2-oxo-4,5-diméthyl-5-éthyl-3-thiazoline-O-(méthylcarbamoyl)-oxime ou d'un sel de ce composé formé par addition avec un acide, et un véhicule inerte.

6. Produit pesticide selon la revendication 1, caractérisé en ce qu'il contient une quantité efficace de 2-oxo-4,5-diéthyl-5-méthyl-3-thiazoline-O-(méthylcarbamoyl)-oxime ou d'un sel de ce composé formé par addition avec un acide, et un véhicule inerte.

7. Produit pesticide selon la revendication 1, caractérisé en ce qu'il contient une quantité efficace d'un composé de formule

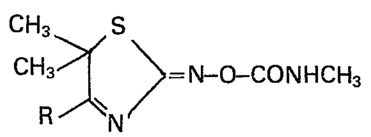 Ia

dans laquelle R représente un groupe éthyle ou isopropyle.

8. Produit pesticide selon la revendication 1, caractérisé en ce qu'il contient une quantité efficace d'un composé de formule

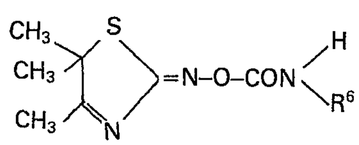 Ib

dans laquelle $R^6$ représente un groupe éthyle, isopropyle, cyclopropyle ou cyclobutyle.

9. Procédé de préparation des composés de formule I de la revendication 1 et de leurs sels, caractérisé en ce que l'on fait réagir une oxime de formule

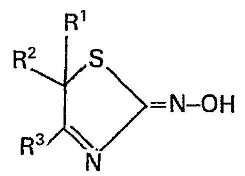 II

dans laquelle $R^1$, $R^2$ et $R^3$ ont les significations indiquées dans la revendication 1,

a) avec un isocyanate de formule

$$R^4-N=C=O \quad III$$

dans laquelle $R^4$ a la signification indiquée dans la revendication 1, en présence d'un diluant et le cas échéant en présence d'un catalyseur, ou bien

b) avec un halogénure de carbamoyle de formule

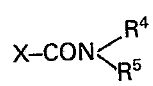 IV

dans laquelle $R^4$ et $R^5$ ont les significations indiquées dans la revendication 1 et X représente un atome d'halogène, en présence d'un diluant et d'un agent fixant les acides, et si on le désire, on convertit un composé obtenu, répondant à la formule I, à l'aide d'un acide, en un sel formé par addition avec l'acide.

10. Utilisation de l'un des composés mentionnés dans les revendications 1 à 8 dans la lutte contre les parasites.